# EUROPEAN PATENT SPECIFICATION

(11) **EP 3 500 316 B1**
(45) Date of publication and mention of the grant of the patent: **01.11.2023**
(21) Application number: 17841739.0
(22) Date of filing: 17.08.2017
(51) Int. Cl.: A61M 1/00, A61F 5/44, A61G 9/02

(54) **A SYSTEM FOR COLLECTING URINE**
SYSTEM ZUR ENTNAHME VON URIN
SYSTÈME DE COLLECTE D'URINE

(30) Priority: 19.08.2016 MY PI2016703037
(43) Date of publication of application: 26.06.2019
(73) Proprietor: Singh, Gurmit, Selangor Darul Ehsan 47640 (MY)
(72) Inventor: Singh, Gurmit, Selangor Darul Ehsan 47640 (MY)
(74) Representative: Potter Clarkson
(86) International application number: PCT/MY2017/000030
(87) International publication number: WO 2018/034558

(56) References cited:
- WO-A1-03/055423
- JP-A- 2004 242 779
- US-A- 5 466 229
- US-A1- 2010 010 286
- US-B1- 6 526 603
- US-B1- 8 394 073

## Description

### FIELD OF INVENTION

The present invention generally relates to a human bodily waste collector and particularly to a urine collector. The present invention more particularly relates to a portable system for collecting and disposing urine from urine bags, which further collect urine directly from a patient.

### BACKGROUND OF THE INVENTION

A urine collector is important especially in healthcare facilities that handle patients with in-dwelling urinary catheters that are affected with a variety of diseases. Conventionally, a healthcare personnel will drain urine from the urine bag attached to the patient and collect the urine before manually disposing the urine in a toilet.

Urinary drainage bags are conventionally used in hospitals and health care facilities when it is necessary to collect urine from a catheterized patient over an extended period of time. Such bags are routinely used by post-operative patients as well as those with urological, neurological, orthopaedic and other disorders for collection, measuring, and testing the frequency of urinary output. Urinary drainage systems typically include a catheter with tubing attached thereto, connected to a collection bag made of a polymeric material such as a PVC film. The collection bag generally includes a component for emptying the bag, such as a drainage tube. In operation, the patient is first catheterized, and the catheter is then connected to the drainage bag through a length of tubing. The bag is normally supported either from the bed rail or other support structure (usually below the level of the patient), the urine draining through the catheter, the tubing, and then finally into the bag due to gravitational forces. Most bags are provided with drain ports through which measured quantities of urine can be removed for various testing procedures.

One of the potential drawbacks with typical urine collection bags is the possibility of contamination and infection to the patient. In particular, when the bags are opened to remove some or all of the urine, air may be permitted to enter into the outlet spout, allowing bacteria to migrate up the spout into the bag, and finally into the bladder, causing infection. Additionally, there may be the problem of contamination of hospital personnel or floor due to leakage or splattering of urine during the collection process. Urine may also collect in the tubing connecting the catheter to the urine collection bag, referred to as urine stasis or hold up within the tubing. While collecting urine from the bags in such vessels as jugs, urine is agitated and thus spreads odour.

US Patent 20140338764 discloses a portable fluid collection apparatus for collecting fluid produced by a human and US Patent US2004187200A1 describes a urine collection device wherein the device comprises of a stand to support the pump, receptacle and reservoir. The prior arts are not removably attached to a urine bag and does not mention the use of a power source to facilitate pump movement. The prior arts are not capable of storing large amount of urine in the collection device.

Other methods include container cups. The urine collection container cups come in a variety of shapes and sizes with lids that can either be snapped on or screwed on. Some urine transport cup closures have special access ports that allow dosed-system transfer of urine directly from the collection device to the tube. However, the containers are not convenient as the healthcare personnel would have to transport the container cups indiscreetly to the toilet, empty them before coming back to collect from another patient especially if the healthcare facility has a large number of patients in each ward. Moreover, the beds are usually far apart from toilets. Apart from that, the expelling process of urine from the urine bag to the container cups will take long as it depends on the gravitational force. The disposal of the urine from the container cup into the toilet will also cause splattering. The urine splatter can reach the eyes, any open wounds and exposed skin of the healthcare personnel or soil the floor of the toilet. Thus, such methods are, indiscreet, dangerous, inconvenient and difficult to be implemented and used by healthcare facilities especially wards with high risk patients.

US patent application no. 20080312550 provide a multifunctional and modular urine collection system comprising urine collection devices (including bags), systems of collecting urine, methods of making and using urine collection bags, and kits comprising urine collection bags. But the prior art fails to provide the adequate disposal of the collected urine from the urine bags. Further the prior art fails to provide a discreet, safe, hygienic, time-saving and energy-saving manner without exposing healthcare personnel to health risks of handling urine.

US patent no. 6,526,603 discloses a system that allows a disabled person in a wheelchair to automatically empty the contents of a bodily waste collection receptacle, such as a leg bag, without the aid of an attendant.

EP 2 359 879 A2 discloses a waste collection unit, for collecting waste material during a series of medical procedures, comprising multiple waste containers and a transfer valve between the waste containers.

In view of the foregoing, there is a need to develop a portable urine collection and disposal system that comprises of a closed system wherein urine can be collected and disposed in a discreet, safe, hygienic, time-saving and energy-saving manner without exposing the healthcare personnel to health risks of handling urine.

### SUMMARY OF THE INVENTION

Thus, the primary object of the present invention, as set out in the appended set of claims, is to provide a urine collecting system, wherein the urine is collected from urine bags.

Another object of the present invention is to provide a portable urine collecting system for a proper disposal of the collected urine without contaminating the heath care personnel.

Yet another object of the present invention is to provide a portable urine collecting system that is capable of transporting the collected urine from the place of collection to the place of disposal without spillage of the collected urine.

Yet another object of the present invention is to provide a urine collecting system that is capable of collecting urine from a plurality of bags in a consecutive manner.

Yet another object of the present invention is to dispose of the collected urine, collected via motor, forcefully thus reducing the time of collection and disposal activity of urine.

The embodiments of the present invention provide a portable system for collecting liquid bodily waste from a bodily waste collecting bag to an indwelling catheter to drain urine from a patient hygienically, comprises: a reservoir for collecting liquid bodily waste from the plurality of patients; an inlet, wherein the inlet is connected with the bodily waste collecting bag; a pumping means comprising an inlet pump to facilitate the movement of the liquid bodily waste into the reservoir and a discharge pump for discharging the urine to an outlet from the reservoir; wherein an inflatable balloon (37) is present on the reservoir (14) to compensate any pressure change inside the reservoir (14); and wherein a urine discharge channel (46) is pulled out from an outer covering (45) at the time of discharging the urine from the reservoir (14).

According to an embodiment of the present invention, the pumping means (16) comprises an inlet pump (35) and a discharge pump (36). The urine is discharged from the outlet (12) from the reservoir (14) with the help of discharge pump (36).

According to an embodiment of the present invention, the handle (32) is bendable backward up to a degree of angle, wherein the angle is at least (30) degrees.

According to an embodiment of the present invention, the portable system comprises a rechargeable battery (44) for running the system without any electric power source or a retractable charging cord.

According to an embodiment of the present invention, the portable system comprises a fresh water tank 40, wherein the fresh water tank is openable through an opening, wherein the opening is covered with a fresh water tank cap 41. An antiseptic or disinfectant or a bactericidal may be added to the fresh water tank.

According to an embodiment of the present invention, the portable system is covered with an outer covering (45).

According to an embodiment of the present invention, the portable system is placed on a base (33).

According to an embodiment of the present invention, the urine collecting system further comprises a brake, a space for holding items, a module for recording the logs of patients and an LCD display.

These and other aspects of the embodiments herein will be better appreciated and understood when considered in conjunction with the following description and the accompanying drawings. It should be understood, however, that the following descriptions, while indicating preferred embodiments and numerous specific details thereof, are given by way of illustration and not of limitation.

### BRIEF DESCRIPTION OF THE DRAWINGS

Other objects, features, and advantages of the invention will be apparent from the following description when read with reference to the accompanying drawings. In the drawings, wherein like reference numerals denote corresponding parts throughout the several views:
**FIG. 1** is a schematic of the urine collecting system showing the various components, according to an embodiment of the present invention.
**FIG. 2** is a perspective view of the urine collecting system, according to an embodiment of the present invention.
**FIG. 3** is a side perspective view of the urine collecting system, according to an embodiment of the present invention.
**FIG. 4** is a perspective view of the urine collecting system showing a housing or cover (**45)**, according to an embodiment of the present invention.
**FIG. 5** is a rear perspective view showing an outlet discharge channel (46), according to an embodiment of the present invention.

### DETAILED DESCRPTION OF THE PREFERRED EMBODIMENTS

The present invention will now be described in detail with reference to the accompanying drawings.

The various embodiments of the present invention provide a urine collecting system comprising various components working together to collect urine or any liquid bodily waste from plurality or multiple bags of various patients, and storing, and disposing the same. The urine collecting system is placed over a base or platform having a plurality of wheels and a handle for movement.

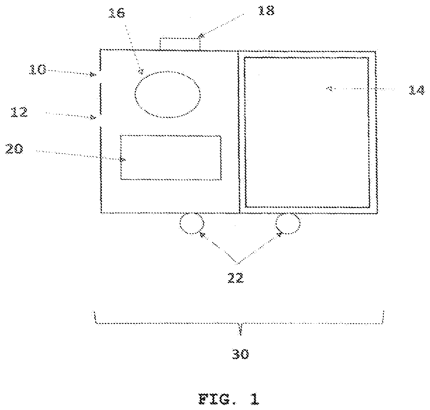

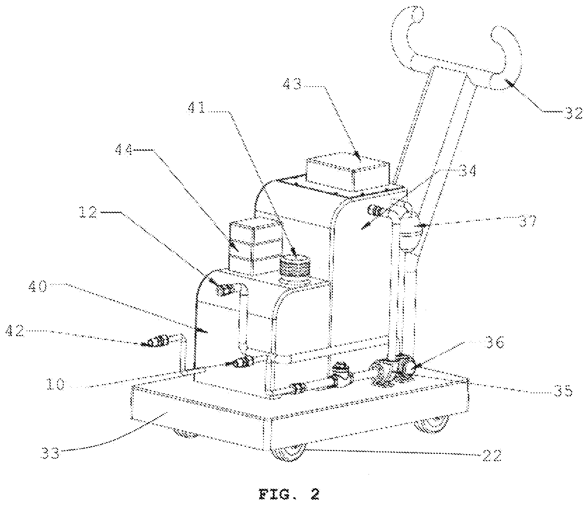

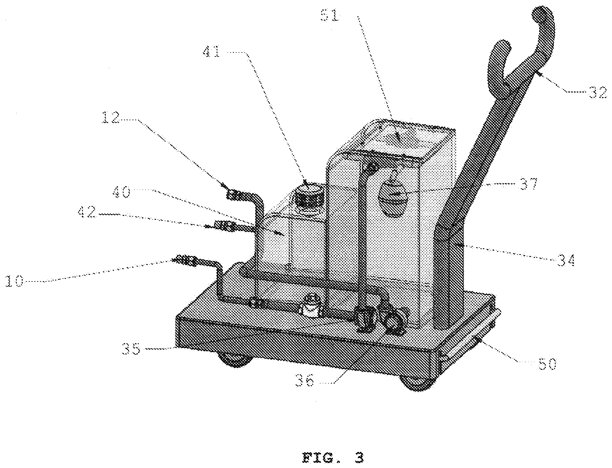

FIGs. 1 to 5 show different perspective views of the urine collecting system from urine bags, according to an embodiment of the present invention. With respect to **FIG. 1** to **FIG. 5****,** a urine collecting system (**30)** comprises an inlet (**10)** which connects with an outlet (12) of a urine bag through a reservoir (**14)** to store urine. A pumping means (**16)** is connected with the inlet (**10)** to facilitate the movement of urine into the reservoir (**14)** and from the reservoir (**14)**, an outlet (**12)** adapted for disposal of the urine from the reservoir (**14).** A power source (**20)** connected with the pumping means (**16)** to control operation of the urine collector (**30)** wherein the pumping means (**16)** draws the urine from the urine bag through the inlet (**10)** into the reservoir (**14)** and forces out the urine from the reservoir (**14)** through the outlet (**12)** transported to and from the disposing location and provide a force strong enough to draw and expel the urine as fast as possible, thus saving time.

The whole system is arranged on a platform or base (**33)** comprising at least a pair of wheels (**22).** The urine collecting system (**30)** further comprises a urine tank (**34)** to temporarily collect and store the urine. The urine is collected inside the urine tank (**34)** through the inlet tube (**10)** wherein the urine is circulated from the inlet tube (**10)** to the urine tank (**34)** with the help of an inlet pump (**35).** An inflatable balloon (**37)** is present on the urine tank to compensate any pressure change inside the urine tank (**34).** The balloon (**37)** is made up of a rubber like material.

The urine is discharged from the outlet (**12)** from the urine tank (**34)** with the help of discharge pump (**36)**.

The pumping means (**16)** comprises an inlet pump (**35)** and a discharge pump (**36).** The inlet pump and outlet pump can be combined or one pump can be used based on the mechanism of peristaltic pumping or alternative polarity.

A fresh water tank (**40)** is provided on the base or platform (**33)** for storing a fresh water. A fresh water tank cap (**41)** is provided on the fresh water tank (**40)** which can be opened to fill in the tank (**40)** with fresh water. The fresh water is stored for flushing. The flushing is done through a fresh water inlet/outlet (**42).** The flush cycle cleans and disinfects the whole system. The fresh water pump or cycle starts either after the urine is flushed from the reservoir (14) or as soon as urine level in the reservoir (14) reaches a predetermined level while getting drained. In an embodiment, a disinfectant or a bactericidal or an antiseptic is added to the water tank.

The inlet tube (**10)** is same for fresh water tank (**40)** and the urine tank (**34),** according to an embodiment of the present invention.

The urine collector system (**30)** further comprises a handle **(32)** to help in easy movement of the urine collecting system. The handle (**32)** is bent-backward type for ease of movement. The handle is bendable up to a certain degree of angle, wherein the angle is at least (30) degrees. A control unit (**43)** is provided to control the operations of the urine collecting system (**30).** A rechargeable battery (**44)** is also provided on the urine collecting system for running the system without any electric power source.

A UV light source is provided in the urine tank (**34)** to disinfect the collected urine. The UV rays kill the microorganisms and disinfect the urine. This retards the proliferation/growth of odour causing bacteria.

A sprinkler system (**51)** is present over the reservoir 14. In an embodiment, the sprinkler system is a hollow tube with a plurality of perforation and the urine is received in the tube from the bag connected to in-dwelling catheter of the patient, and the urine falls to base of the reservoir via the perforations, thus increasing the exposure of urine drops to UV rays.

According to an embodiment of the present invention, the urine collector system (**30)** is able to draw, store and expel urine that is collected from the urine bags. In an embodiment, a light source is attached to the system.

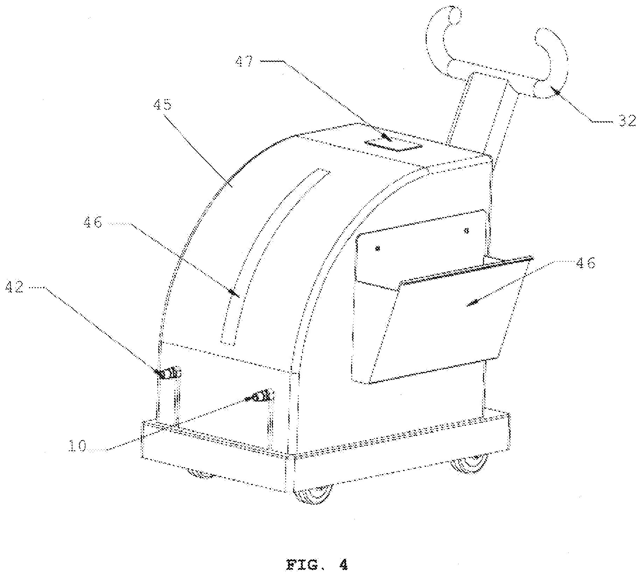

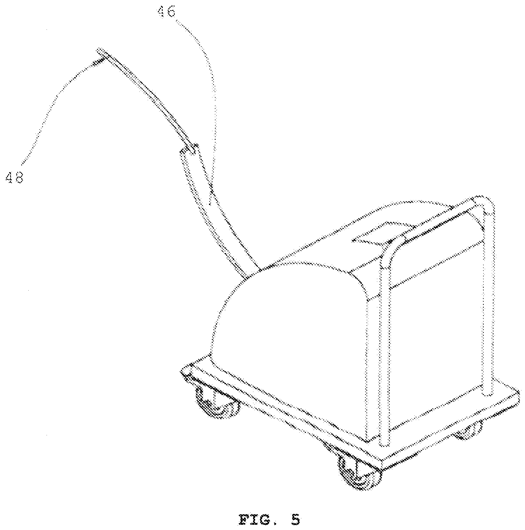

**FIG. 4** shows a housing or cover (**45)** present over the urine collection system. The housing (**45)** comprises urine discharge channel (**46)** that is pulled out from the housing (**45)** at the time of discharging the urine as shown in **FIG. 5****.** An outlet tube (**48)** is detached from the track and flapped outward to direct into toilet for discharging the urine.

The urine collector (**30)** further comprises at least one wheel (**22)** and more likely to have a pair of wheels or more in order to facilitate movement around the healthcare facility and to the disposal area such as the toilet. A brake/ lock wheels (**50)** is provided on the backside or frontside lower end to control the movement of the system. A space (**46)** is present to store items like files, gloves, or any miscellaneous items. The space (**46)** is present over the housing of the system. The configuration and structure of the present invention may vary but does not go beyond the essence of the present invention.

According to an embodiment herein, the urine collecting system further comprises a module for recording the logs i.e. the details of urine collection is stored in a memory. The details comprise patient name, time of collection, volume of urine, etc. The records are shown on an LCD display (**47)** present over the urine collection system.

Thus, the present invention provide a portable and movable system that is capable of collecting a huge amount of urine from a multiple urine bags belonging to multiple patients, to dispose of the urine hygienically.

As will be readily apparent to those skilled in the art, the present invention may easily be produced in other specific forms without departing from its essential characteristics. The present embodiments is, therefore, to be considered as merely illustrative and not restrictive, the scope of the invention being indicated by the claims.

## Claims

1. A portable system for collecting liquid bodily waste from a bodily waste collecting bag connected to an indwelling catheter to drain urine from a patient hygienically, comprises:
a reservoir (14) for collecting liquid bodily waste from the patient;
an inlet (10), wherein the inlet is connected with the bodily waste collecting bag;
a pumping means (16) comprising an inlet pump (35) to facilitate the movement of the liquid bodily waste into the reservoir (14) and a discharge pump (36) for discharging the urine to an outlet (12) from the reservoir (14);
wherein an inflatable balloon (37) is present on the reservoir (14) to compensate any pressure change inside the reservoir (14); and
wherein a urine discharge channel (46) is pulled out from an outer covering (45) at the time of discharging the urine from the reservoir (14).

2. The system as claimed in claim 1, further comprising a fresh water tank (40), connected to the reservoir (14); wherein the fresh water tank flushes the reservoir (14) after or while the urine is drained from the reservoir (14).

3. The system as claimed in claim 1, wherein the bodily waste is urine.

4. The system as claimed in claim 1 further comprising a power source (20); and a control unit (43).

5. The system as claimed in claim 1 further comprising UV light source in the reservoir (14).

6. The system as claimed in claim 1, wherein the portable system comprises a rechargeable battery (44) for running the system without any electric power source.

7. The system as claimed in claim 1, wherein the portable system is placed on a base (33).

8. The system as claimed in claim 1, wherein the portable system further comprises a brake, a space for holding items, a module for recording the logs of the patient and an LCD.

## Patentansprüche

1. Tragbares System zum Sammeln von flüssigen Körperausscheidungen aus einem Körperausscheidungssammelbeutel, der mit einem Dauerkatheter verbunden ist, um Urin von einem Patienten hygienisch abzuleiten, umfasst:
ein Reservoir (14) zum Sammeln der flüssigen Körperausscheidungen des Patienten;
einen Einlass (10), wobei der Einlass mit dem Körperausscheidungssammelbeutel verbunden ist;
ein Pumpmittel (16), das eine Einlasspumpe (35) umfasst, um die Bewegung der flüssigen Körperausscheidungen in das Reservoir (14) zu erleichtern, und eine Auslasspumpe (36) zum Auslassen des Urins zu einem Auslass (12) aus dem Reservoir (14);
wobei ein aufblasbarer Ballon (37) auf dem Reservoir (14) vorhanden ist, um jede Druckänderung innerhalb des Reservoirs (14) auszugleichen; und
wobei ein Urinabflusskanal (46) zur Zeit des Abflusses des Urins aus dem Reservoir (14) aus einer äußeren Abdeckung (45) herausgezogen wird.

2. System nach Anspruch 1, das ferner einen Frischwassertank (40) umfasst, der mit dem Reservoir (14) verbunden ist; wobei der Frischwassertank das Reservoir (14) spült, nachdem oder während der Urin aus dem Reservoir (14) abgelassen wird.

3. System nach Anspruch 1, wobei es sich bei den Körperausscheidungen um Urin handelt.

4. System nach Anspruch 1, das ferner eine Leistungsquelle (20) und eine Steuereinheit (43) umfasst.

5. System nach Anspruch 1, das ferner eine UV-Lichtquelle in dem Reservoir (14) umfasst.

6. System nach Anspruch 1, wobei das tragbare System eine wiederaufladbare Batterie (44) umfasst, um das System ohne elektrische Leistungsquelle zu betreiben.

7. System nach Anspruch 1, wobei das tragbare System auf eine Basis (33) gestellt wird.

8. System nach Anspruch 1, wobei das tragbare System ferner eine Bremse, einen Raum zur Aufnahme von Gegenständen, ein Modul zur Aufzeichnung der Patientenprotokolle und ein LCD umfasst.

## Revendications

1. Système portable pour collecter des déchets corporels liquides à partir d'un sac de collecte de déchets corporels relié à un cathéter à demeure pour drainer l'urine d'un patient de manière hygiénique, comprend :
un réservoir (14) pour collecter des déchets corporels liquides du patient ;
une entrée (10), l'entrée étant reliée au sac de collecte de déchets corporels ;
un moyen de pompage (16) comprenant une pompe d'entrée (35) pour faciliter le mouvement des déchets corporels liquides dans le réservoir (14) et une pompe de refoulement (36) pour évacuer l'urine vers une sortie (12) du réservoir (14) ;
dans lequel un ballon gonflable (37) est présent sur le réservoir (14) pour compenser tout changement de pression à l'intérieur du réservoir (14) ; et
dans lequel un canal d'évacuation d'urine (46) est retiré d'un revêtement extérieur (45) au moment de l'évacuation de l'urine du réservoir (14).

2. Système selon la revendication 1, comprenant en outre un réservoir d'eau douce (40), relié au réservoir (14) ; dans lequel le réservoir d'eau douce rince le réservoir (14) après ou pendant que l'urine est évacuée du réservoir (14).

3. Système selon la revendication 1, dans lequel les déchets corporels sont de l'urine.

4. Système selon la revendication 1, comprenant en outre une source d'alimentation (20) ; et une unité de commande (43).

5. Système selon la revendication 1, comprenant en outre une source de lumière UV dans le réservoir (14).

6. Système selon la revendication 1, dans lequel le système portable comprend une batterie rechargeable (44) pour faire fonctionner le système sans aucune source d'énergie électrique.

7. Système selon la revendication 1, dans lequel le système portable est placé sur un socle (33).

8. Système selon la revendication 1, dans lequel le système portable comprend en outre un frein, un espace pour contenir des objets, un module pour enregistrer les journaux du patient et un LCD.
